# EUROPEAN PATENT SPECIFICATION

(11) **EP 1 513 791 B1**
(45) Date of publication and mention of the grant of the patent: **15.06.2011**
(21) Application number: 03733858.9
(22) Date of filing: 16.04.2003
(51) Int. Cl.: C07C 41/00, C07C 2/02, C07C 2/56, B01D 3/34, B01D 3/00

(54) **PROCESS AND APPARATUS FOR CATALYTIC DISTILLATIONS**
VERFAHREN UND VORRICHTUNG FÜR KATALYTISCHE DESTILLIERUNGEN
PROCEDE ET APPAREIL DE DISTILLATION CATALYTIQUE

(30) Priority: 11.06.2002 US 167196
(43) Date of publication of application: 16.03.2005
(73) Proprietor: Catalytic Distillation Technologies, Pasadena, Texas 77507 (US)
(72) Inventor: GROTEN, Willibrord, A., Pasadena, TX 77507 (US); MARASCHINO, Mario, J., Houston, TX 77242-2833 (US)
(74) Representative: Zumstein, Angela
(86) International application number: PCT/US2003/011654
(87) International publication number: WO 2003/104168

(56) References cited:
- WO-A-02/33027
- US-A- 5 362 377
- US-A- 5 811 597
- US-A- 5 919 989
- US-B1- 6 232 509
- US-B1- 6 335 473

## Description

### BACKGROUND OF THE INVENTION

### Field of the Invention

The present invention relates to an apparatus and the method for carrying out catalytic distillations using a divided wall column, for example the etherification of isoolefins, particularly C₅ isoolefins with methanol to produce the corresponding tertiary ether, wherein catalytic distillation is used in a divided wall catalytic distillation reactor to simultaneously separate tertiary amyl methyl ether (TAME) and react substantially all of the methanol to preclude the use of a separate methanol recovery system.

### Related Information

A divided wall distillation column or divided wall column is a distillation vessel having a vertical partition separating one side from the other for a portion or all of the height of the vessel. The divided wall column may have a common rectification section, a common stripping section or both. Such divided wall columns are variously described in U.S. Patent Nos. 4,230,533; 4,582,569; 4,826,574; 5,339,648 and 5,755,933. Engineering design methods are assumed to be used to assure proper distribution of upflowing vapor to the alternate sides of a divided-wall column. Such methods to control vapor split may be active or passive. Also, engineering design methods are assumed to assure the proper controlled split of the liquid to both sides of a divided wall device. Such splits are purposely targeted to accomplish specific design objectives as determined from rigorous simulation analysis of the intended operation.

A specialized use of a distillation column, known as catalytic distillation has been used in etherifications, hydrogenations, hydrodesulfurizations, isomerizations, thioetherifications, oligomerizations and others. The catalytic distillation process employs a catalyst system (see U.S. Patent Nos. 4,215,011 and 4,302,356) which provides for both reaction and distillation concurrently in the same reactor, at least in part within the catalyst system. The method involved is briefly described as one where concurrent reaction and distillation occur in a combination reactor-distillation structure as described in several U.S. Patents, namely U.S. Patent Nos. 4,242,530; 4,250,052; 4,232,177; 4,302,356; 4,307,254; and 4,336,407.

The reaction of an alcohol and an olefin and concurrent separation of the reactants from the reaction products by fractional distillation has been practiced for some time. The process is variously described in several of the previously cited patents and U.S. Patent Nos. 4,504,687; 4,987,807; and 5,118,873.

As an example, in a catalytic distillation etherification system the alcohol and isoolefin are fed to a distillation column reactor having a distillation reaction zone containing suitable catalyst, such as an acid cation exchange resin, preferably in the form of catalytic distillation structure, and also preferably, having a distillation zone containing an inert distillation structure, e.g., trays, saddles, and the like. As embodied in the etherification of iC₄⁼'s and/or iC₅⁼'s the olefin and an excess of methanol may be first fed to a straight pass reactor wherein most of the olefin is reacted to form the corresponding ether, methyl tertiary butyl ether (MTBE) or tertiary amyl methyl ether (TAME). The feeds may contain both normal and iso olefins. The reaction is highly selective toward the isoolefins. The straight pass reactor is preferably operated at a given pressure such that the reaction mixture is at the boiling point, thereby limiting the temperature rise across the reactor by permitting the exothermic heat of reaction to partially vaporize the mixture. A straight pass reactor and process are described more completely in U.S. Patent No. 4,950,803 which is hereby incorporated by reference.

The effluent from the first reactor is then fed to a distillation column reactor wherein the unreacted isoolefins are converted to ether, the excess methanol and unreacted hydrocarbons are withdrawn as an overhead product while the ether is withdrawn as bottoms product stream.

As noted above, in the etherification of olefins with an alcohol there is preferably an excess of the alcohol available. This excess alcohol is typically recovered from the overhead stream in downstream units.

In the case of the C₅'s system the overhead product will contain the azeotropic level of MeOH which is about 12 wt%. If the net flow of methanol into the column (allowing for that reacting in the column) is less than the azeotrope concentration in the distillate, the methanol concentration in the reaction distillation zone will be relatively quite low, about 1 %. If the net methanol flow into the column is higher than the azeotrope, the methanol concentration will increase (60% has been measured) until methanol leaves with the TAME bottoms product. Neither case is desirable because at low concentration the conversion of isoamylene to TAME is low whereas at high concentrations the TAME purity is affected by the presence of the excess methanol.

The methanol feed is thus best controlled to produce the highest methanol concentration within the catalyst bed while preventing methanol leaving with the bottoms product. This results in close to the azeotropic concentration in the distillate product and in the reaction distillation zone. The methanol must be separated from the hydrocarbons so that the hydrocarbons can be used for gasoline blending and to conserve methanol. The separation is usually achieved by washing the hydrocarbon/methanol mixture with water. The methanol is selectively absorbed in the water phase which is subsequently fractionated to separate the methanol.

The recovery of the methanol requires considerable amounts of water energy and significant number of theoretical stages which substantially increases the operating and capital cost of the process. It is an advantage of the present invention that in an etherification embodiment wherein an alcohol azeotrope is formed an alcohol recovery section is not required. It is a further advantage of the present invention that the alcohol/hydrocarbon azeotrope is maintained throughout essentially all of the reaction distillation zone, maximizing conversion of the reactive olefins.

### SUMMARY OF THE INVENTION

Briefly, the present invention is a distillation column having at least two vertical distillation sections, at least one of said sections containing catalyst, preferably in a form to serve as distillation structure, and at least one of said sections being free of catalyst and the process of concurrently carrying out the reactions of a material with itself or other materials to produce products thereof and fractional separation of the product and the starting materials therein. The sections are separated by a wall extending through a vertical portion of the distillation column. The vertical portion of the separating wall comprises less than the total height of the column. The vertical wall preferably extends across the lateral dimension of the column and may extend vertically to either the top or to the bottom of the column or to neither. The sections are in fluid communication around a vertical terminus either at the upper end or the bottom end of the vertical wall or both. Thus the present apparatus provides an integrated distillation and catalytic distillation system.

The apparatus of the present invention may be characterized as a catalytic distillation column having three internal sections, at least one of said sections containing catalyst, two of said sections being separated by a vertical wall extending through a portion of said catalytic distillation column, said parallel sections being in communication above and/or below said vertical wall. Preferably, the sections include a common rectification section above the vertical wall and two parallel sections which are a simple distillation section and the other contains a catalytic distillation zone. In a further embodiment, a common stripping section is included below the vertical wall to the catalytic distillation column.

The present process may be used for reacting a first component with itself or a second component to produce a product and comprises: (a) feeding a first material comprising a first component or said first component and a second component to a distillation column reactor; (b) concurrently: (1) contacting said first component or first component and said second component with a catalytic distillation structure in a distillation reaction zone thereby catalytically reacting at least a portion of said first component with itself or said first and second components to form a product and (2) fractionating a first mixture comprising said first component and said product or said first component, said second component and said product; and (c) withdrawing product from the distillation column reactor; wherein the improvement comprises concurrently with (a) and (b) in said distillation reaction column: contacting a second mixture comprising said first component and said product or said first component, said second component and said product with a non catalytic distillation structure in a parallel and separate distillation non reaction zone to fractionate said third component product and withdrawing said third component product from said distillation non reaction zone.

In a C₅ etherification the first section is operated under conditions of temperature and pressure to separate any ether in the feed to the column and to fractionate hydrocarbons, including any unreacted isoolefins overhead with any alcohol as an azeotrope. The alcohol is consumed in the second section or removed from the overhead condensate.

For the purposes of the present invention, the term "catalytic distillation" includes any process of concurrent reaction and fractional distillation in a column regardless of the designation applied thereto. Several different arrangements have been disclosed to achieve the desired result. For example, British Patents 2,096,603 and 2,096,604 disclose placing the catalyst on conventional trays within a distillation column. A series of U.S. patents, including particularly U.S. Patent Nos. 4,443,559 and 4,215,011, exemplify using the catalyst as part of the packing in a packed distillation column.

### BRIEF DESCRIPTION OF THE DRAWING

FIG. 1 is a schematic representation of the present apparatus used for the synthesis of a TAME according to present invention.
FIG. 2 is configuration of the present apparatus having a common stripping section in addition to a common rectification section.

### DETAILED DESCRIPTION OF THE PREFERRED EMBODIMENTS

The use of a divided wall distillation column in a catalytic distillation is not heretofore described in the art. The feed to the present divided wall catalytic distillation column reactor may be from a prior reactor in which less than all of the reactants were reacted. The feed material contains the reactants and product which is fed to a non reaction distillation section to separate the product and allow the reactants to be further reacted in the catalytic distillation section to produce more product. Makeup material may be added as required. The present divided wall catalytic distillation column reactor may also be used as the primary reactor in which the reactants are fed to the catalytic distillation section and fractionated in both vertical sections. There may be a common rectification section above the divided vertical sections or a common stripping section below or both.

The operation of the present invention is described for etherification, however, the use of the divided wall column is also suitable for the other reactions, including those presently carried out under catalytic distillation conditions.

In the reactions the first component may react with itself, such as the production of a dimer from the reaction of olefin with itself or with a second component such as the reaction of an olefin with an alcohol.

Among the suitable reactions are:
oligomerization of olefins such as dimerization and the reaction of the dimers with olefins or other dimers of single olefins or mixtures of olefins, such as the oligomerization of isobutene;
reaction of alcohols to produce diethers such as the reaction of methanol with itself to form dimethyl ether ;
etherification of olefins with alcohols to produce ethers;
thioetherification of dienes with mercaptans to produce sulfides;
skeletal isomerization of olefins with or without hydrogen;
position isomerization of olefins with or without hydrogen:
   reaction of mercaptans and thiophenes with hydrogen to produce H₂S;
   reaction of acetylenes, dienes ,olefin or mixtures with hydrogen;
   reaction of cyclic olefins and aromatic compounds with hydrogen;
   reaction of aromatic compounds and alkane derivative compounds with ammonia to form amines;
   reaction of nitriles with a hydration compound;
   reaction of acetone with hydrogen to form methyl ethyl ketone;
   reaction of aromatic compounds with olefins to form alkylated aromatic compounds;
   reaction of carbon monoxide with hydrogen to methanol and the like.

In carrying out the some of the processes, it will be appreciated that additional feed lines may be necessary. For example, in those reactions in which hydrogen or ammonia are present in the reaction zone, a feed is provided below the catalyst bed, preferably within the catalyst section. In a preferred operation the reactants, e.g. alcohol and olefin, are maintained within the column by feeding a sufficient amount of a lower boiling inert material which forms the overhead and reflux to the system to the exclusion of the reactants.

A catalytic distillation process employs a catalyst system (See U.S. Patent Nos. 4,215,011 and 4,302,356) which provides for both reaction and distillation concurrently in the same reactor, at least in part, within the catalyst system. The method involved is briefly described as one where concurrent reaction and distillation occur in a combination of reactor-distillation structures which are described in several U.S. Patents, namely U.S. Patent Nos. 4,242,530; 4,250,052; 4,232,177; 4,302,356; 4,307,254; and 4,336,407 which are incorporated herein in their entirety. Additionally U.S. Patent Nos. 4,302,356 and 4,443,559 disclose catalyst structures which are useful as distillation structures.

For example, methanol and isoamylene (or the stream from the boiling point reactor which contains, ether, some unreacted isoolefin and methanol or make up methanol) containing C₅ stream are continuously fed to the distillation column reactor where they are contacted in the catalytic distillation structure. The methanol preferentially reacts with isoamylene, forming TAME which is heavier than the C₅ components of the feed and the methanol, hence it drops in the column to form the bottoms. Concurrently, the unreacted C₅'s (e.g., n-pentane, n-pentenes) are lighter and form an overhead. The olefins in the feeds to the reaction usually contain linear and branched olefins, e.g., n-butenes, isobutene, n-amylenes and isoamylenes. The alcohols are preferably monohydric, such as methanol, ethanol, propanol and mixtures thereof. The branched chained olefins are selectively more reactive and the reactions may be operated to favor their reaction, particularly the tertiary olefins as known in the art.

In a further embodiment having a common rectification section, an extractive solvent may be added during the reactions to the distillation column reactor to any or all sections of the column at a location above the feed but below the upper terminus of the incoming side and/or below the upper terminus but within or above a catalyst bed. It is understood that the extractive solvent would be at least 25°C higher boiling than the product of the reaction and that extractive solvent would be at least 60°C higher boiling than the first component described hereinabove. The extractive solvent should comprise at least 50 volume % of the internal liquid in order to be effective at altering relative volatilities in the column. Preferably the extractive solvent may be recovered and separated from the extracted material for recycle within the system.

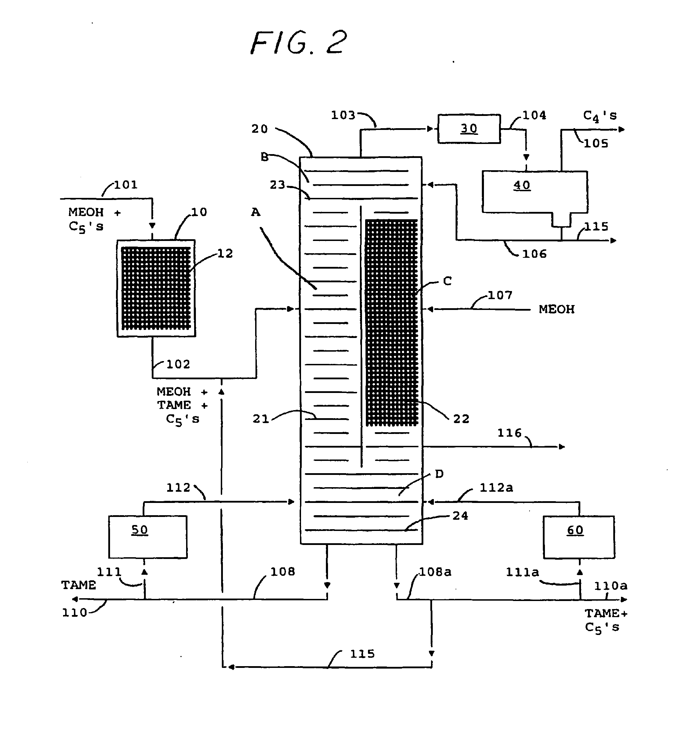

Referring now to the drawings, an etherification carried out in the present apparatus operates generally as follows:
Section A distills ether in feed from unreacted alcohol and isolefins in feed. Non-reactive hydrocarbons that are more volatile than the ether are also removed from the ether. Vapor exits the top of the section A and enters the common rectification section B. Reflux to section A is provided from common rectification section B. The division of the reflux as to how much goes to zone "A" versus how much goes to zone "C" is intentionally designed for and is to be controlled.
Section B rectification section concentrates light components in feed that are purged as overhead product. Purging of lights is needed to control temperatures in catalytic distillation columns. Section B also controls the ether product level in purge to a very low level (ppm), and minimizes the loss of C₅'s to the overheads.
Section C parallels section A and is fed via liquid reflux from the bottom of section B and vapor from stripping section D (if present) or reboiler (if section D is not present). If stripping section D is utilized (as in Figure 2), the split of the vapor between side "A" and side "C" is intentionally controlled at prescribed levels using either active or passive engineering-design means. Catalytic distillation converts alcohol and isoolefin to ether in section C. Trays below the catalytic distillation zone strip MeOH from hydrocarbons and ether by using its azeotrope with the hydrocarbons. A stripping section "D" can be utilized as shown in Figure 2. If so, a sidedraw 116 from section "C" below the catalyst bed but above the bottom of the divide can be utilized to pull off C₅'s with some accompanying TAME. It is advantageous to draw off the vapor as a sidedraw via conduit 116, and condense it downstream (not shown). This allows an essentially near C₅-free pure TAME product to be withdrawn from the bottom of the unit as stream 110a with minimal diverting of product TAME to the sidedraw conduit 116.

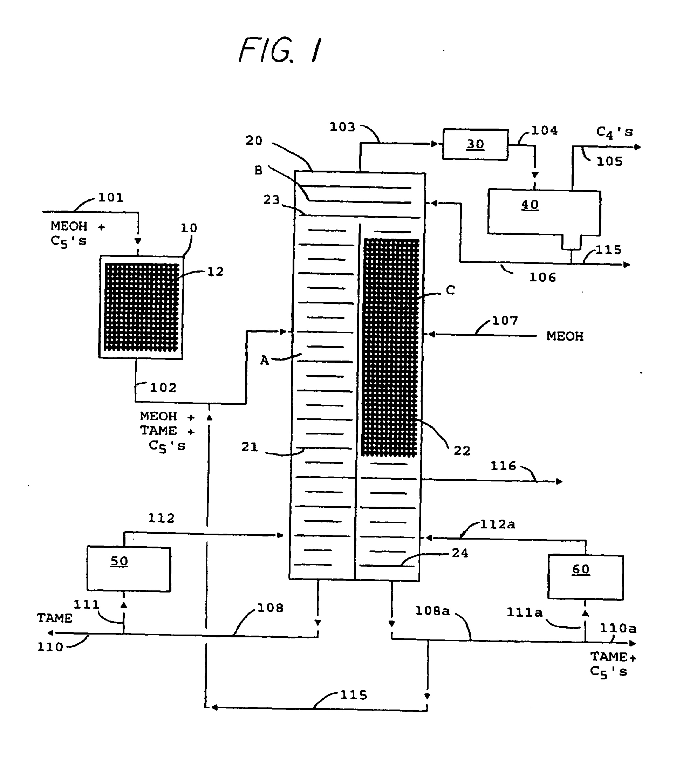

FIG. 1 shows a simplified flow diagram of a TAME process utilizing the present invention. The divided wall distillation column reactor 20 generally includes three sections: section A) containing a first distillation zone (stripping section) which contains inert distillation structure at the bottom to separate the TAME from unreacted methanol, reactive C₅ hydrocarbons and inerts; section C) a middle reaction zone containing the catalytic distillation structure, where the etherification occurs; and section B) an upper distillation zone (rectification section) containing inert distillation structure to separate back into the reaction zone any unreacted isoamylenes and some methanol. As noted methanol and C₅'s form an azeotrope. This azeotrope boils about 10 to 15°F lower than the C₅'s and is thus predominantly in the upper distillation zone and overheads.

In a conventional distillation column reactor there is generally a reflux of condensed overheads to facilitate the separation of the more volatile unreacted components from the product. In the case of etherification of C₅ olefins with methanol to produce TAME, the overheads usually contain methanol, inert C₅'s (i.e., normal pentenes or pentanes) and other lighter inert materials which might be in the feed. The condensible overheads are recovered and the methanol is usually separated from the hydrocarbons as by water washing, the methanol being selectively removed in the water phase. The methanol and water may then be separated by distillation and the methanol recycled to the reactor.

The present divided wall column presents another solution. The condensed reflux containing methanol and C₅ olefins, which is comprised of the overheads from both sides of the column, descends into both sides of the column. In a first side the reflux without catalyst simply helps separate the product TAME from the unreacted methanol and C₅'s. However, in a second side, catalyst is loaded and the methanol and C₅ olefins react to produce additional TAME. The second side is operated to separate the methanol/C₅ azeotrope from the TAME/C₅ mixture. In the preferred mode, the reactor is operated "dead headed in methanol". This means that substantially all of the methanol entering the catalytic distillation unit is destined to be consumed by reaction with very little methanol (if any) leaving in any of the column effluent streams. Preferably, the total reactive-methanol requirements required by both fixed-bed prereactor and by the catalytic distillation reactor enter through conduit 101. This will allow for better conversion within the fixed bed prereactor, and satisfy the reactive stoichiometric requirements for the catalytic distillation column. Composition monitoring within section "C" is desirable to keep the methanol inventory in the column in good balance with the reactive needs. The total methanol feed rate to the reactive system is adjusted so as to maintain the methanol profile at near azeotrope composition across the catalyst yet avoid pushing methanol out of the lowermost conduits of the system so as to essentially keep methanol away from any withdrawn TAME-rich product. The net effect of the present invention is to integrate the benefits from both the catalytic distillation column and the divided wall column.

Catalysts preferred for the etherification process are resin cation exchangers, which contain sulfonic acid groups, and which have been obtained by polymerization or copolymerization of aromatic vinyl compounds followed by sulfonation. The resulting products preferably contain an average of 1.3 to 1.8 sulfonic acid groups per aromatic nucleus. Particularly, suitable polymers which contain sulfonic acid groups are copolymers of aromatic monovinyl compounds with aromatic polyvinyl compounds, particularly, divinyl compounds, in which the polyvinyl benzene content is preferably 1 to 20% by weight of the copolymer (see, for example, German Patent specification 908,247). The ion exchange resin is preferably used in a granular size of about 0.25 to 1 mm, although particles from 0.15 mm up to about 2 mm may be employed.

A preferred catalytic distillation structure for use herein comprises placing the cation exchange resin particles into porous containers which are surrounded by open space comprising 50-95 volume % of the structure. This allows the requisite flows and prevents loss of catalyst. Suitable structures are described in U.S. Patent Nos. 5,266,546, 4,731,229, 5,073,236, 5,266,546, 5,431,890 and 5,730,843 which are incorporated by reference. The catalytic distillation structure when loaded into the column constitutes a distillation reaction zone.

Referring to FIG. 1, methanol and mixed C₅'s containing isoamylenes are fed via flow line 101 to the primary reactor 10 containing a bed of catalyst 12. The resin catalyst is loaded into the straight pass reactor 10 as a fixed bed of granules. The feed to the reaction is fed to the bed in liquid phase. U.S. Patent Nos. 5,003,124 and 4,950,803 which are incorporated herein, disclose a liquid phase process for the etherification of C₄ to C₆ isoolefins with C₁ to C₆ alcohols in a boiling point straight pass reactor (boiling point reactor) that is controlled at a pressure to maintain the reaction mixture at its boiling point and where the effluent may be fed directly to a catalytic distillation reactor. The bed may be horizontal, vertical or angled with either upflow or downflow of the reactants and reaction products. Preferably the bed is vertical with the feed passing downward through the bed and exiting, after reaction, through the lower end of the reactor. In the reactor 10 a portion of the isoamylenes reacts with methanol to form tertiary amyl methyl ether (TAME) which exits the reactor 10 as effluent via flow line 102 along with unreacted methanol and C₅'s.

The effluent from the reactor in flow line 102 is fed into section A of a divided wall column. Section A comprises a zone 21 containing standard distillation structure such as sieve tray, bubble cap tray or packing. In the zone 21 the TAME is separated from unreacted methanol and C₅'s. The TAME is taken as bottoms via flow line 108 some of which is recycled through reboiler 50 via flow lines 111 and 112. TAME product is taken via flow line 110. Note that in the configuration shown as FIG. 2, the reboiler 50 and reboiler 60 may be combined into a single reboiler. Product streams 108 and 108a become as merged. Also as merged are streams 112 together with 112a. Finally, product stream 110 also becomes merged with stream 110a.

In all cases, the vapor traveling upwards in the column is purposely divided at the lowermost terminus of the dividing wall in a prescribed ratio as determined beforehand from rigorous reactive-distillation simulation of divided-wall configuration. Such division of flow may be controlled to prescribed values by engineering-design methods incorporating either active or passive means. Similarly, the liquid traveling down the column approaching the upper terminus is purposely divided as well in a manner consistent with goals established from same said reactive distillation simulation design exercises. Again, such division of flow is held at prescribed values using engineering design methods incorporating either active or passive means. These considerations are understood to be so in all cases.

In the section B the unreacted methanol and C₅'s are rectified in common rectification zone 23 to concentrate lighter boiling components such as C₃ and C₄ hydrocarbons in the vapor phase which are taken as overheads via flow line 103 and passed to partial condenser 30 and are then passed on to separator 40 via flow line 104. The lights are purged either as vapor via flow line 105 or as condensed liquid via flow line 115 or both. A portion the condensed liquid (including C₅'s and methanol) is fed back as reflux to common rectification section 23 via conduit 106. The removal of light components from zone 23 is important for controlling the temperature in section C.

Section C operates in parallel to section A and contains a catalytic distillation zone 22 where a portion of the unreacted isoamylenes react further with methanol to form additional TAME. Additional methanol to meet catalytic distillation reaction needs is co-fed way upstream within stream 101 or (optionally) co-fed via flow line 107 or a combination of both. The methanol feed rate is adjusted to maintain a near azeotropic composition across the bed. However, excess methanol beyond this feed rate which would result in methanol loss to product is to be avoided. In the distillation zone 22 below the catalyst bed but above the terminus working together and contiguously with the distillation zone 24, the unreacted methanol and isoamylenes are stripped from the the TAME/C₅ mixture which is taken as bottoms via flow line 108a. Some of the TAME/C₅ mixture is recycled through reboiler 60 via flow lines 111 a and 112a. A product containing TAME and C₅'s is withdrawn via flow line 110a.

In summary the TAME product is separated from the methanol/C₅ azeotrope in section A, lights are removed from methanol/C₅ in section B and TAME and C₅'s are separated from the methanol/C₅'s in section C while reacting methanol with isoamylenes in the second side. Because only enough methanol is added to make up for that reacted and to support the azeotropes in the system, there is no need for additional process equipment normally associated with separating methanol from C₅ mixtures.

Fig. 2 shows an integrated distillation and catalytic distillation system similar to Fig.1 except that a common stripping section D replaces the two separate stripping sections and a single bottoms is recovered.

As methanol previously, the bulk of the C₅s accompanied by some TAME can (optionally) be taken as a sidedraw 116 as illustrated in both FIG. 1 and FIG. 2. A vapor sidedraw is preferred as it can be richer in C₅s and leaner in TAME. Said vapor drawoff may be condensed in an external condenser and knockout pot which is routed to product storage. With such configuration, it becomes possible to recover an essentially pure TAME stream as a bottoms product.

## Claims

1. A process for reacting a first component alone or with a second component to produce a product comprising:
(a) feeding a first material comprising a first component or said first component and a second component to a distillation column reactor;
(b) concurrently :
(1) contacting said first component or said first component and said second component with a catalytic distillation structure in a distillation reaction zone thereby catalytically reacting at least a portion of said first component with itself or with said second component to form a product and
(2) fractionating a first mixture comprising said first component and said product or said first component, said second component and said product; and
(c) withdrawing the product from the distillation column reactor;
wherein the improvement comprises concurrently with (a) and (b) in said distillation column reactor:
contacting a second mixture comprising said first component and said product or said first component, said second component and said product with a non catalytic distillation zone in a parallel and separate distillation non reaction zone to fractionate said product and withdrawing said product from said distillation non reaction zone.

2. The process according to claim 1 comprising the oligomerization of olefins.

3. The process according to claim 1 comprising the etherification of olefins with alcohols.

4. The process according to claim 1 comprising the thioetherification of dienes with mercaptans.

5. The process according to claim 1 comprising skeletal isomerization of olefins.

6. The process according to claim 1 comprising the position isomerization of olefins.

7. The process according to claim 1 comprising the reaction of mercaptans, thiophenes or mixtures thereof with hydrogen.

8. The process according to claim 1 comprising the reaction of dienes, olefin or mixtures thereof with hydrogen.

9. The process according to claim 1 comprising the reaction of cyclic olefins with hydrogen.

10. The process according to claim 1 comprising the reaction of aromatic compounds with hydrogen.

11. The process according to claim 1 comprising the reaction of nitriles with a hydration compound.

12. The process according to claim 1 comprising the reaction of aromatic compounds with ammonia.

13. The process according to claim 1 comprising the reaction of aromatic compounds with olefin.

14. The process according to claim 1, wherein the catalytic distillation structure is a fixed bed acidic cation exchange resin packing.

15. The process according to claim 14, wherein
(a) the first material comprises a C4 to C7 olefin or mixtures thereof and a C1 to C6 alcohol or mixtures thereof;
(b) the product is an ether product,
(c) the second material comprises a C4 to C7 olefin or mixtures thereof and a C1 to C6 alcohol or mixtures thereof and an ether product.

16. The process according to claim 15, wherein said second mixture comprises olefin and alcohol corresponding to (a) and ether product corresponding to (b).

17. The process according to claim 16, wherein said second mixture is fed to said distillation column reactor and a portion of said first material is derived from said second material.

18. The process according to claim 16, wherein said first material is fed to the distillation column reactor and second mixture is derived from the reaction of said first material.

19. The process according to claim 17, wherein alcohol is added to said distillation reaction zone.

20. The process according to claim 15, wherein ether product is separately withdrawn from said distillation reaction zone and said distillation non reaction zone.

21. The process according to claim 15, wherein unreacted materials are withdrawn together from said distillation reaction zone and said distillation non reactive zone at a point above said feeding.

22. The process according to claim 15, wherein said olefins comprise C5 olefins and said stream contains inert hydrocarbon diluent having a boiling point lower than the ether product.

23. The process according to claim 22, wherein said olefins comprise isobutene.

24. The process according to claim 22, wherein said olefins comprise a mixture of isobutene and n-butenes.

25. The process according to claim 22, wherein said diluent comprises C4 alkane.

26. The process according to claim 15, wherein said olefins comprise C5 olefins and said stream contains inert hydrocarbon diluent having a boiling point lower than the ether product.

27. The process according to claim 26, wherein said olefins comprise isoamylene.

28. The process according to claim 26, wherein said olefins comprise a mixture of isoamylene and n-amylenes.

29. The process according to claim 15, wherein said alcohol has one hydroxyl group.

30. The process according to claim 29, wherein said alcohol is methanol, ethanol, propanol or mixtures thereof.

31. The process according to claim 29, wherein said alcohol comprises ethanol.

32. The process according to claim 29, wherein said alcohol comprises propanol.

33. The process according to claim 29, wherein said alcohol comprises a mixture of ethanol and methanol.

34. The process according to claim 24, wherein said contacting and reacting of olefins and alcohol are carried out to react substantially only isobutene with said alcohol.

35. The process according to claim 15, wherein said alcohol forms an azeotrope with an olefin component in said process.

36. The process according to claim 15, wherein an inert component having a lower boiling point than the lowest boiling olefin component in said process is present in said process to provide an overhead component and reflux thereby excluding olefin and alcohol from said overhead.

37. The process according to claim 28, wherein said contacting and reacting of olefins and alcohol are carried out to react substantially only isoamylene with said alcohol.

38. The process according to claim 15, wherein said olefins are isoolefins.

39. The process according to claim 38, wherein said alcohols are primary alcohols.

40. The process according to claim 1, wherein
said first component comprises C4 to C7 isoolefins or a mixture thereof, primary alcohol having one to six carbon atoms or mixtures thereof and the ethers corresponding thereto, and
said second component comprises a primary alcohol having one to six carbon atoms, and
said catalytic reaction comprises reacting
at least a portion of said olefins and said primary alcohol to form said product, wherein said product is an ether product, and
said fractionating comprises fractionating at least a portion of said ether product from at least a portion of unreacted primary alcohol; and wherein
said withdrawing comprises withdrawing the ether from said integrated distillation and catalytic distillation system at a point below said feeding, and
withdrawing a portion of unreacted materials from said integrated distillation and catalytic distillation system at a point above said feeding.

41. The process according to claim 40, wherein unreacted hydrocarbons and ether are withdrawn together from said integrated distillation and catalytic distillation system.

42. The process according to claim 40, wherein said olefins comprise C5 olefins and said stream contains inert hydrocarbon diluent having a boiling point lower than the ether product.

43. The process according to claim 40, wherein said olefins comprise isobutene.

44. A distillation column having at least two vertical distillation sections, at least one of said sections containing catalyst and at least one of said sections being free of catalyst, said sections being separated by a wall extending through a vertical portion of said distillation column, said vertical portion comprising less than the total height of said column and said sections being in fluid communication around a vertical terminus of said wall.

45. The distillation column according to claim 44 comprising a common rectification section above said vertical sections.

46. The distillation column according to claim 44 comprising a common stripping section below said vertical sections.

47. The distillation column according to claim 44 comprising a common rectification section above said vertical sections and a common stripping section below said vertical sections.

48. The distillation column according to claim 44 comprising a sidedraw below said catalyst-containing section.

49. The distillation column according to claim 45 having means to divide a liquid leaving the common rectification section to alternative vertical distillation sections at predetermined ratios.

50. The distillation column according to claim 46 having means to divide a vapor leaving the common stripping section to alternative vertical distillation sections at predetermined ratios.

51. The distillation column according to claim 45 having means to add an extractive solvent to at least one of said sections, below said upper terminus.

52. The distillation column according to claim 49 wherein said means to add an extractive solvent is above a feed.

53. The distillation column according to claim 49 wherein said means to add an extractive solvent is within said catalyst.

54. The distillation column according to claim 49 wherein said means to add an extractive solvent is above said catalyst

## Patentansprüche

1. Verfahren zum Umsetzen einer ersten Komponente allein oder mit einer zweiten Komponente zur Herstellung von einem Produkt, umfassend:
(a) Zuführen eines ersten Materials, umfassend eine erste Komponente oder die erste Komponente und eine zweite Komponente, zu einem Destillations-Kolonnen-Reaktor;
(b) gleichzeitig:
(1) In-Kontakt-Bringen der ersten Komponente oder der ersten Komponente und der zweiten Komponente mit einer katalytischen Destillations-Struktur in einer Destillations-Reaktions-Zone, wodurch katalytisch mindestens ein Teil von der ersten Komponente mit sich selbst oder mit der zweiten Komponente zur Bildung von einem Produkt umgesetzt wird und
(2) Fraktionieren von einem ersten Gemisch, umfassend die erste Komponente und das Produkt oder die erste Komponente, die zweite Komponente und das Produkt; und
(c) Abziehen von dem Produkt aus dem Destillations-Kolonnen-Reaktor;
wobei die Verbesserung gleichzeitig mit (a) und (b) in dem Destillations-Kolonnen-Reaktor umfasst:
In-Kontakt-Bringen von einem zweiten Gemisch, umfassend die erste Komponente und das Produkt oder die erste Komponente, die zweite Komponente und das Produkt, mit einer nicht-katalytischen Destillations-Zone in einer parallelen und getrennten Destillations-Nicht-Reaktions-Zone zum Fraktionieren des Produkts und Abziehen des Produkts aus der Destillations-Nicht-Reaktions-Zone.

2. Verfahren nach Anspruch 1, umfassend die Oligomerisierung von Olefinen.

3. Verfahren nach Anspruch 1, umfassend die Veretherung von Olefinen mit Alkoholen.

4. Verfahren nach Anspruch 1, umfassend die Thioveretherung von Dienen mit Mercaptanen.

5. Verfahren nach Anspruch 1, umfassend Gerüst-Isomerisierung von Olefinen.

6. Verfahren nach Anspruch 1, umfassend die Positions- bzw. Stellungs-Isomerisierung von Olefinen.

7. Verfahren nach Anspruch 1, umfassend die Reaktion von Mercaptanen, Thiophenen oder Gemischen davon mit Wasserstoff.

8. Verfahren nach Anspruch 1, umfassend die Reaktion von Dienen, Olefin oder Gemischen davon mit Wasserstoff.

9. Verfahren nach Anspruch 1, umfassend die Reaktion von cyclischen Olefinen mit Wasserstoff.

10. Verfahren nach Anspruch 1, umfassend die Reaktion von aromatischen Verbindungen mit Wasserstoff.

11. Verfahren nach Anspruch 1, umfassend die Reaktion von Nitrilen mit einer Hydratations-Verbindung.

12. Verfahren nach Anspruch 1, umfassend die Reaktion von aromatischen Verbindungen mit Ammoniak.

13. Verfahren nach Anspruch 1, umfassend die Reaktion von aromatischen Verbindungen mit Olefin.

14. Verfahren nach Anspruch 1, wobei die katalytische Destillations-Struktur eine saure Kat-ionen-Austausch-Harz-Festbett-Packung ist.

15. Verfahren nach Anspruch 14, wobei
(a) das erste Material ein C4 bis C7-Olefin oder Gemische davon und einen C1 bis C6-Alkohol oder Gemische davon umfasst;
(b) das Produkt ein Ether-Produkt ist,
(c) das zweite Material ein C4 bis C7-Olefin oder Gemische davon und einen C1 bis C6-Alkohol oder Gemische davon und ein Ether-Produkt umfasst.

16. Verfahren nach Anspruch 15, wobei das zweite Gemisch Olefin und Alkohol entsprechend (a) und Ether-Produkt entsprechend (b) umfasst.

17. Verfahren nach Anspruch 16, wobei das zweite Gemisch zu dem Destillations-Kolonnen-Reaktor gespeist wird und ein Teil von dem ersten Material von dem zweiten Material abgeleitet ist.

18. Verfahren nach Anspruch 16, wobei das erste Material zu dem Destillations-Kolonnen-Reaktor gespeist wird und das zweite Gemisch von der Reaktion von dem ersten Material abgeleitet ist.

19. Verfahren nach Anspruch 17, wobei ein Alkohol zu der Destillations-Reaktions-Zone gegeben wird.

20. Verfahren nach Anspruch 15, wobei das Ether-Produkt getrennt aus der Destillations-Reaktions-Zone und der Destillations-Nicht-Reaktions-Zone abgezogen wird.

21. Verfahren nach Anspruch 15, wobei nicht umgesetzte Materialien zusammen aus der Destillations-Reaktions-Zone und der Destillations-Nicht-reaktiven Zone an einem Punkt oberhalb der Zuspeisung abgezogen werden.

22. Verfahren nach Anspruch 15, wobei die Olefine C5-Olefine umfassen und der Strom inertes Kohlenwasserstoff-Verdünnungsmittel mit einem Siedepunkt niedriger als das Ether-Produkt enthält.

23. Verfahren nach Anspruch 22, wobei die Olefine Isobuten umfassen.

24. Verfahren nach Anspruch 22, wobei die Olefine ein Gemisch von Isobuten und n-Butenen umfassen.

25. Verfahren nach Anspruch 22, wobei das Verdünnungsmittel C4-Alkan umfasst.

26. Verfahren nach Anspruch 15, wobei die Olefine C5-Olefine umfassen und der Strom inertes Kohlenwasserstoff-Verdünnungsmittel mit einem Siedepunkt niedriger als das Ether-Produkt enthält.

27. Verfahren nach Anspruch 26, wobei die Olefine Isoamylen umfassen.

28. Verfahren nach Anspruch 26, wobei die Olefine ein Gemisch von Isoamylen und n-Amylenen umfassen.

29. Verfahren nach Anspruch 15, wobei der Alkohol eine Hydroxyl-Gruppe aufweist.

30. Verfahren nach Anspruch 29, wobei der Alkohol Methanol, Ethanol, Propanol oder Gemische davon ist.

31. Verfahren nach Anspruch 29, wobei der Alkohol Ethanol umfasst.

32. Verfahren nach Anspruch 29, wobei der Alkohol Propanol umfasst.

33. Verfahren nach Anspruch 29, wobei der Alkohol ein Gemisch von Ethanol und Methanol umfasst.

34. Verfahren nach Anspruch 24, wobei das In-Kontakt-Bringen und Umsetzen von Olefinen und Alkohol ausgeführt werden, um im Wesentlichen nur Isobuten mit dem Alkohol umzusetzen.

35. Verfahren nach Anspruch 15, wobei der Alkohol ein Azeotrop mit einer Olefin-Komponente in dem Verfahren bildet.

36. Verfahren nach Anspruch 15, wobei eine inerte Komponente mit einem niedrigeren Siedepunkt als die am niedrigsten siedende Olefin-Komponente in dem Verfahren in dem Verfahren vorliegt, um eine Overhead-Komponente und Rückfluss bereitzustellen, wodurch Olefin und Alkohol von dem Overhead ausgeschlossen werden.

37. Verfahren nach Anspruch 28, wobei das In-Kontakt-Bringen und Umsetzen von Olefinen und Alkohol ausgeführt werden, um im Wesentlichen nur Isoamylen mit dem Alkohol umzusetzen.

38. Verfahren nach Anspruch 15, wobei die Olefine Isoolefine sind.

39. Verfahren nach Anspruch 38, wobei die Alkohole primäre Alkohole sind.

40. Verfahren nach Anspruch 1, wobei
die erste Komponente C4 bis C7-Isoolefine oder ein Gemisch davon, primären Alkohol mit einem bis sechs Kohlenstoff-Atomen oder Gemische davon und die dazu entsprechenden Ether umfasst, und
die zweite Komponente einen primären Alkohol mit einem bis sechs Kohlenstoff-Atomen umfasst,
und
die katalytische Reaktion umfasst Umsetzen von mindestens einem Teil von den Olefinen und dem primären Alkohol, um das Produkt zu bilden, wobei das Produkt ein Ether-Produkt ist, und
das Fraktionieren Fraktionieren von mindestens einem Teil von dem Ether-Produkt von mindestens einem Teil von nicht umgesetztem primärem Alkohol umfasst; und wobei das Abziehen Abziehen des Ethers aus dem integrierten Destillations- und katalytischen Destillations-System an einem Punkt unterhalb der Zuspeisung, und
Abziehen eines Teils von nicht umgesetzten Materialien aus dem integrierten Destillations- und katalytischen Destillations-System an einem Punkt oberhalb der Zuspeisung umfasst.

41. Verfahren nach Anspruch 40, wobei nicht umgesetzte Kohlenwasserstoffe und Ether zusammen aus dem integrierten Destillations- und katalytischen Destillations-System abgezogen werden.

42. Verfahren nach Anspruch 40, wobei die Olefine C5-Olefine umfassen und der Strom inertes Kohlenwasserstoff-Verdünnungsmittel mit einem Siedepunkt niedriger als das Ether-Produkt enthält.

43. Verfahren nach Anspruch 40, wobei die Olefine Isobuten umfassen.

44. Destillations-Kolonne mit mindestens zwei vertikalen Destillations-Abschnitten, wobei mindestens einer von den Abschnitten Katalysator enthält und mindestens einer von den Abschnitten frei von Katalysator ist, wobei die Abschnitte durch eine Wand getrennt sind, die sich über einen vertikalen Teil von der Destillations-Kolonne erstreckt, wobei der vertikale Teil weniger als die Gesamt-Höhe von der Kolonne umfasst und die Abschnitte sich in fluider Kommunikation um einen vertikalen Terminus bzw. Abschluss von der Wand befinden.

45. Destillations-Kolonne nach Anspruch 44, umfassend einen gemeinsamen Rektifikations-Abschnitt oberhalb der vertikalen Abschnitte.

46. Destillations-Kolonne nach Anspruch 44, umfassend einen gemeinsamen Abstreif-Abschnitt unterhalb der vertikalen Abschnitte.

47. Destillations-Kolonne nach Anspruch 44, umfassend einen gemeinsamen Rektifikations-Abschnitt oberhalb der vertikalen Abschnitte und einen gemeinsamen Abstreif-Abschnitt unterhalb der vertikalen Abschnitte.

48. Destillations-Kolonne nach Anspruch 44, umfassend einen Seitenabzug unterhalb des Katalysator-enthaltenden Abschnitts.

49. Destillations-Kolonne nach Anspruch 45 mit Mitteln zum Teilen einer Flüssigkeit, die den gemeinsamen Rektifikations-Abschnitt verlässt, zu alternativen vertikalen Destillations-Abschnitten bei vorbestimmten Verhältnissen.

50. Destillations-Kolonne nach Anspruch 46 mit Mitteln zum Teilen eines Dampfs, der den gemeinsamen Abstreif-Abschnitt verlässt, zu alternativen vertikalen Destillations-Abschnitten bei vorbestimmten Verhältnissen.

51. Destillations-Kolonne nach Anspruch 45 mit Mitteln zum Hinzu-Fügen eines extraktiven Lösungsmittels zu mindestens einem von den Abschnitten unterhalb des oberen Terminus bzw. Abschlusses.

52. Destillations-Kolonne nach Anspruch 49, wobei das Mittel zum Hinzu-Fügen eines extraktiven Lösungsmittels sich oberhalb einer Zuspeisung befindet.

53. Destillations-Kolonne nach Anspruch 49, wobei das Mittel zum Hinzu-Fügen eines extraktiven Lösungsmittels sich in dem Katalysator befindet.

54. Destillations-Kolonne nach Anspruch 49, wobei das Mittel zum Hinzu-Fügen eines extraktiven Lösungsmittels sich oberhalb des Katalysators befindet.

## Revendications

1. Procédé pour faire réagir un premier constituant seul ou avec un second constituant pour produire un produit, comprenant les étapes suivantes :
(a) acheminer un premier matériau comprenant un premier constituant ou ledit premier constituant et un second constituant à un réacteur à colonne de distillation ;
(b) simultanément :
(1) mettre en contact ledit premier constituant ou ledit premier constituant et ledit second constituant avec une structure de distillation catalytique dans une zone réactionnelle de distillation, faisant ainsi réagir catalytiquement au moins une partie dudit premier constituant avec lui-même ou avec ledit second constituant pour former un produit, et
(2) fractionner un premier mélange comprenant ledit premier constituant et ledit produit ou ledit premier constituant, ledit second constituant et ledit produit ; et
(c) retirer le produit du réacteur à colonne de distillation ;
le perfectionnement consistant, parallèlement aux étapes (a) et (b) dans ledit réacteur à colonne de distillation, à :
mettre en contact un second mélange comprenant ledit premier constituant et ledit produit ou ledit premier constituant, ledit second constituant et ledit produit avec une zone de distillation non catalytique dans une zone non réactionnelle de distillation parallèle et séparée pour fractionner ledit produit et retirer ledit produit de ladite zone non réactionnelle de distillation.

2. Procédé selon la revendication 1, comprenant l'oligomérisation d'oléfines.

3. Procédé selon la revendication 1, comprenant l'éthérification d'oléfines avec des alcools.

4. Procédé selon la revendication 1, comprenant la thioéthérification de diènes avec des mercaptans.

5. Procédé selon la revendication 1, comprenant l'isomérisation squelettique d'oléfines.

6. Procédé selon la revendication 1, comprenant l'isomérisation de position d'oléfines.

7. Procédé selon la revendication 1, comprenant la réaction de mercaptans, thiophènes ou leurs mélanges avec de l'hydrogène.

8. Procédé selon la revendication 1, comprenant la réaction de diènes, oléfines ou leurs mélanges avec de l'hydrogène.

9. Procédé selon la revendication 1, comprenant la réaction d'oléfines cycliques avec de l'hydrogène.

10. Procédé selon la revendication 1, comprenant la réaction de composés aromatiques avec de l'hydrogène.

11. Procédé selon la revendication 1, comprenant la réaction de nitriles avec un composé d'hydratation.

12. Procédé selon la revendication 1, comprenant la réaction de composés aromatiques avec de l'ammoniac.

13. Procédé selon la revendication 1, comprenant la réaction de composés aromatiques avec de l'oléfine.

14. Procédé selon la revendication 1, dans lequel la structure de distillation catalytique est une garniture de résine échangeuse de cations acide à lit fixe.

15. Procédé selon la revendication 14, dans lequel
(a) le premier matériau comprend une oléfine en C4-C7 ou ses mélanges et un alcool en C1-C6 ou ses mélanges ;
(b) le produit est un produit d'éther,
(c) le second matériau comprend une oléfine en C4-C7 ou ses mélanges et un alcool en C1-C6 ou ses mélanges et un produit d'éther.

16. Procédé selon la revendication 15, dans lequel ledit second mélange comprend une oléfine et un alcool correspondant à (a) et un produit d'éther correspondant à (b).

17. Procédé selon la revendication 16, dans lequel ledit second mélange est alimenté au réacteur à colonne de distillation et une partie dudit premier matériau est dérivée dudit second matériau.

18. Procédé selon la revendication 16, dans lequel ledit premier matériau est alimenté au réacteur à colonne de distillation et le second mélange est dérivé de la réaction dudit premier matériau.

19. Procédé selon la revendication 17, dans lequel l'alcool est ajouté à ladite zone réactionnelle de distillation.

20. Procédé selon la revendication 15, dans lequel le produit d'éther est retiré séparément de ladite zone réactionnelle de distillation et de ladite zone non réactionnelle de distillation.

21. Procédé selon la revendication 15, dans lequel des matériaux n'ayant pas réagi sont retirés ensemble de ladite zone réactionnelle de distillation et de ladite zone non réactionnelle de distillation en un point situé au-dessus de ladite alimentation.

22. Procédé selon la revendication 15, dans lequel lesdites oléfines comprennent des oléfines en C5 et ledit flux contient un diluant d'hydrocarbure inerte ayant un point d'ébullition inférieur à celui du produit d'éther.

23. Procédé selon la revendication 22, dans lequel lesdites oléfines comprennent de l'isobutène.

24. Procédé selon la revendication 22, dans lequel lesdites oléfines comprennent un mélange d'isobutène et de n-butènes.

25. Procédé selon la revendication 22, dans lequel ledit diluant comprend de l'alcane en C4.

26. Procédé selon la revendication 15, dans lequel lesdites oléfines comprennent des oléfines en C5 et ledit flux contient un diluant d'hydrocarbure inerte ayant un point d'ébullition inférieur à celui du produit d'éther.

27. Procédé selon la revendication 26, dans lequel lesdites oléfines comprennent de l'isoamylène.

28. Procédé selon la revendication 26, dans lequel lesdites oléfines comprennent un mélange d'isoamylène et de n-amylènes.

29. Procédé selon la revendication 15, dans lequel ledit alcool présente un groupe hydroxyle.

30. Procédé selon la revendication 29, dans lequel ledit alcool est du méthanol, de l'éthanol, du propanol ou leurs mélanges.

31. Procédé selon la revendication 29, dans lequel ledit alcool comprend de l'éthanol.

32. Procédé selon la revendication 29, dans lequel ledit alcool comprend du propanol.

33. Procédé selon la revendication 29, dans lequel ledit alcool comprend un mélange d'éthanol et de méthanol.

34. Procédé selon la revendication 24, dans lequel la mise en contact et la mise en réaction d'oléfines et d'alcool sont opérées pour faire réagir quasiment uniquement l'isobutène avec ledit alcool.

35. Procédé selon la revendication 15, dans lequel ledit alcool forme un azéotrope avec un constituant d'oléfine dans ledit procédé.

36. Procédé selon la revendication 15, dans lequel un constituant inerte ayant un point d'ébullition plus bas que le constituant d'oléfine ayant le point d'ébullition le plus bas dans ledit procédé, est présent dans ledit procédé pour fournir un constituant de tête et un reflux, excluant ainsi l'oléfine et l'alcool de ladite tête.

37. Procédé selon la revendication 28, dans lequel la mise en contact et la mise en réaction d'oléfines et d'alcool sont opérées pour faire réagir quasiment uniquement l'isoamylène avec ledit alcool.

38. Procédé selon la revendication 15, dans lequel lesdites oléfines sont des isooléfines.

39. Procédé selon la revendication 38, dans lequel lesdits alcools sont des alcools primaires.

40. Procédé selon la revendication 1, dans lequel
ledit premier constituant comprend des isooléfines en C4-C7 ou un mélange de celles-ci, l'alcool primaire ayant un à six atomes de carbone ou des mélanges de ceux-ci et les éthers associés, et
ledit second constituant comprend un alcool primaire ayant un à six atomes de carbone, et
ladite réaction catalytique consiste à faire réagir
au moins une partie desdites oléfines et dudit alcool primaire pour former ledit produit, ledit produit étant un produit d'éther, et
ledit fractionnement consiste à fractionner au moins une partie dudit produit d'éther à partir d'au moins une partie d'alcool primaire n'ayant pas réagi, et dans lequel
ledit retrait consiste à retirer l'éther dudit système de distillation intégrée et système de distillation catalytique à un point situé au-dessous de ladite alimentation, et
retirer une partie des matériaux n'ayant pas réagi dudit système de distillation intégrée et système de distillation catalytique à un point situé au-dessus de ladite alimentation.

41. Procédé selon la revendication 40, dans lequel les hydrocarbures et l'éther qui n'ont pas réagi sont retirés ensemble dudit système de distillation intégrée et système de distillation catalytique.

42. Procédé selon la revendication 40, dans lequel lesdites oléfines comprennent des oléfines en C5 et ledit flux contient un diluant d'hydrocarbure inerte ayant un point d'ébullition inférieur à celui du produit d'éther.

43. Procédé selon la revendication 40, dans lequel lesdites oléfines comprennent de l'isobutène.

44. Colonne de distillation pourvue d'au moins deux sections de distillation verticales, au moins l'une desdites sections contenant un catalyseur et au moins l'une desdites sections étant exempte de catalyseur, lesdites sections étant séparées par une paroi s'étendant à travers une partie verticale de ladite colonne de distillation, ladite partie verticale ayant une hauteur inférieure à la hauteur totale de ladite colonne et lesdites sections étant en communication fluidique autour d'une extrémité terminale verticale de ladite paroi.

45. Colonne de distillation selon la revendication 44, comprenant une section de rectification commune au-dessus desdites sections verticales.

46. Colonne de distillation selon la revendication 44, comprenant une section de strippage commune au-dessous desdites sections verticales.

47. Colonne de distillation selon la revendication 44, comprenant une section de rectification commune au-dessus desdites sections verticales et une section de strippage commune au-dessous desdites sections verticales.

48. Colonne de distillation selon la revendication 44, comprenant un prélèvement latéral au-dessous de ladite section contenant un catalyseur.

49. Colonne de distillation selon la revendication 45, comprenant des moyens pour diviser un liquide quittant la section de rectification commune vers d'autres sections de distillation verticales à des ratios prédéterminés.

50. Colonne de distillation selon la revendication 46, comprenant des moyens pour diviser une vapeur quittant la section de strippage commune vers d'autres sections de distillation verticales à des ratios prédéterminés.

51. Colonne de distillation selon la revendication 45, comprenant des moyens pour ajouter un solvant extractif à au moins l'une desdites sections, au-dessous de ladite extrémité terminale supérieure.

52. Colonne de distillation selon la revendication 49, dans lequel lesdits moyens pour ajouter un solvant extractif sont situés au-dessus d'une alimentation.

53. Colonne de distillation selon la revendication 49, dans lequel lesdits moyens pour ajouter un solvant extractif sont à l'intérieur dudit catalyseur.

54. Colonne de distillation selon la revendication 49, dans lequel lesdits moyens pour ajouter un solvant extractif sont situés au-dessus dudit catalyseur.
